## Europäisches Patentamt
## European Patent Office
## Office européen des brevets

(11) Publication number: **0 083 140**
**B1**

(12) **EUROPEAN PATENT SPECIFICATION**

(45) Date of publication of patent specification: **11.09.85**

(21) Application number: **82201653.1**

(22) Date of filing: **23.12.82**

(51) Int. Cl.⁴: **C 07 C 37/86, C 07 C 37/56, C 07 C 39/04, C 07 C 39/00**

(54) Process for the decarboxylative oxidation of a benzene monocarboxylic acid.

(30) Priority: **24.12.81 NL 8105833**

(43) Date of publication of application:
**06.07.83 Bulletin 83/27**

(45) Publication of the grant of the patent:
**11.09.85 Bulletin 85/37**

(84) Designated Contracting States:
**DE FR GB IT NL**

(56) References cited:
**FR-A-1 383 531**
**FR-A-1 402 836**
**FR-A-1 552 109**
**FR-A-2 075 660**
**GB-A- 911 246**

(73) Proprietor: **STAMICARBON B.V.**
**Postbus 10**
**NL-6160 MC Geleen (NL)**

(72) Inventor: **Van Geem, Paul C.**
**Constantijn Hugostraat 13**
**NL-6176 BS Spaubeek (NL)**
Inventor: **Schellekens, Ronald M. A. M.**
**Europalaan 60**
**NL-6226 CR Maastricht (NL)**

(74) Representative: **van Golde, Lambertus Maria Gerardus T. et al**
**OCTROOIBUREAU DSM P.O. Box 9**
**NL-6160 MA Geleen (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Description

The invention relates to a process for the decarboxylative oxidation of a benzene monocarboxylic acid or a salt, ester, or anhydride of such an acid (hereinafter collectively referred to for the sake of brevity as 'benzene monocarboxylic acid') in the liquid phase at an elevated temperature by means of a molecular oxygen-containing gas in which process the tar-like product also formed is separated.

It is known (for example from Hydrocarbon Processing, Vol. 43, No. 11, November 1965, pp. 173 f.) that phenols can be prepared by a process of this type. The term 'phenol' as used in this description and in the claims encompasses hydroxybenzene and its functional derivatives. Examples of phenols that can be obtained in this way from the corresponding monocarboxylic acids are:

| | |
|---|---|
| from benzoic acid | — phenol |
| from o-toluic acid | — m-cresol |
| from m-toluic acid | — o- and p-cresol |
| from p-toluic acid | — m-cresol |
| from m-nitrobenzoic acid | — p-nitrophenol |
| from p-nitrobenzoic acid | — m-nitrophenol |
| from p-chlorobenzoic acid | — m-chlorophenol and phenol |
| from p-methoxybenzoic acid | — m-methoxyphenol and phenol |
| from p-phenylbenzoic acid | — m-phenylphenol |
| from 2,4-dimethylbenzoic acid | — 2,5-dimethylphenol |

The carboxylic acids may, for example, be prepared by the catalytic oxidation of a monoalkyl benzene compound with a oxygen-containing gas, using a cobalt salt soluble in the reaction medium as a catalyst.

The reaction is usually carried out in the presence of a copper compound dissolved in the reaction mixture. A suitable copper compound for use as the catalyst in this reaction is the copper(I) or copper(II) salt of the carboxylic acid that is to be converted. This copper salt may be produced in the reaction zone by bringing another copper compound, for example copper(II) salicylate, copper(II) oxide, copper(I) oxide, or metallic copper into the reaction zone. A typical copper concentration is 0.1—5 wt.-%. In addition to copper it is customary to use a cocatalyst, for example a soluble magnesium salt such as magnesium benzoate.

The reaction can be carried out by passing a molecular oxygen containing gas through the molten carboxylic acid or through a solution of the carboxylic acid in an inert solvent. A copper compound is dissolved in the liquid medium. It may be advantageous to pass steam through the medium as well. The molecular oxygen containing-gas may, for example, be air, pure oxygen, or an air-oxygen or air-nitrogen mixture. Suitable inert solvents are, for example, water and liquid carboxylic acids. The oxidation can be carried out at a temperature of, for example, 450—600 K and a pressure of 10—1000 kPa, and preferably 100—300 kPa.

The reaction gases which contain the phenol are discharged from the reaction vessel. The phenol can be separated from these gases and after purification can be used for any desired application.

It is also possible to carry out the phenol-forming reaction in two steps, by first oxidizing benzoic acid in a first step without addition of steam, so that together with phenol product a quantity of phenyl benzoate compound is formed as reaction product, and secondly hydrolyzing the said phenyl benzoate compound into phenol and benzoic acid, for instance by introducing steam, separating off the phenol and returning the benzoic acid to the first stage.

Besides the phenol, this oxidation also yields a tar-like product with a high viscosity. This tar is usually burned.

The known process has a serious disadvantage: the formation of the tar-like product lowers the yield of the reaction.

The aim of the invention is to eliminate this disadvantage. The invention accords to a process for the decarboxylative oxidation of a benzene monocarboxylic acid, preferably benzoic acid, or salt, ester, or anhydride of such an acid in the liquid phase at an elevated temperature by means of a molecular oxygen-containing gas, in which process the tar-like product also formed is separated, the said tar-like product being subjected to a hydrolysis treatment at a temperature of 500—850 K and a pressure of 50—3000 kPa in the presence of a suitable hydrolysis catalyst consisting at least in part of one or more metals from groups IIIB and IVB of the periodic table of elements, including the rare earth metals and the actinides, preferably at a load of 0.01—20 kg of the tar-like product per kg of metal in the catalyst per hour. The suprising result of this hydrolysis treatment is that part of the tar is converted into phenol. A great advantage of this process is that the by-product, which hitherto has been burned as waste, is partially converted into the valuable product phenol, so that the overall yield of the said oxidation is often sharply increased. The hydrolysis treatment is preferably carried out by treating the tar-like product with 0.1—10 kg of water and/or steam per kg of the tar-like product.

The hydrolysis treatment is performed preferably at 600—800 K. The pressure is not critical but because of economical reasons lies between 50 and 3000 kPa, and preferably between 100 and 500 kPa, although a gas pressure greater than 3000 kPa is also possible.

A particularly suitable method is to perform the hydrolysis treatment of the tar-like product in accordance with the invention with steam in a trickle-phase reactor, preferably at 600—800 K.

The metals in the catalyst may be used in the metallic form or as the oxide or some other suitable form, individually or as mixtures. Examples of suitable metals are yttrium, cerium, zirconium, hafnium, and especially thorium. The catalyst is preferably supported by a carrier, which may be made of a conventional carrier material such as silica or alumina.

A catalyst that is particularly suitable for use in the process of the invention consists of thoria on α-alumina as carrier. A catalyst of this type is known from British Patent Specification No. 911,246. The preferred working temperature for a catalyst of this type is 500—850 K. A preferred composition for this catalyst is 20—50 wt-% (with respect to the carrier mass) of thoria on α-alumina. The optimal working temperature for this catalyst is 600—800 K. In this temperature range the catalyst gives a high yield of phenol and maximum conversion.

A suitable method of preparation of the said catalysts is precipitation from an aqueous solution of a soluble metal compound, possibly in the presence of suspended carrier material, or coprecipitation from an aqueous solution of a soluble metal compound and a soluble precursor of the carrier material, for example with an aqueous solution of an alkali metal hydroxide, ammonia, or oxalic acid, followed by calcination of the precipitated product in air at 600—1200 K. The said soluble metal compounds may, for example, be nitrates, sulphates, and/or perchlorates.

Another suitable method of preparation is to impregnate the carrier material with a solution of a metal compound that, upon calcination in air at 600—1200 K, is dissociated thermally, such as a nitrate, a carbonate, and/or an oxalate.

The tar-like product is preferably subjected to a preliminary treatment before the hydrolysis. This preliminary treatment consists of distillation of the tar, preferably with passage of oxygen. The effect of the passage of oxygen is that during the distillation the formation of a precipitate which consists of insoluble metals and metal oxides which originate, for example, from the catalyst components is prevented. The oxygen may be supplied in the form of an oxygen-containing gas such as air, oxygen-enriched air, or pure oxygen. The quantity of oxygen supplied during the distillation is preferably 0.1—1 mole of molecular oxygen per mole of the metal. Preferably, as much benzoic acid as possible is also removed from the tar during the preliminary treatment, preferably by crystallizing it out. The hydrolysis treatment of this distillate is carried out in accordance with the invention preferably in the gas phase at a temperature of preferably 600—800 K.

The process of the invention is particularly suited to the decarboxylative oxidation of benzoic acid to phenol, since the tar formed in the process, after being subjected to the hydrolysis treatment in accordance with the invention, preferably in combination with the said preliminary treatment, yields a reaction mixture from which the phenol may be recovered in a very simple way, for example by distillation.

According to the invention the overall yield of phenol of a decarboxylative oxidation of a benzenemonocarboxylic acids as described above can be considerably increased.

The invention will now be explained further by means of the following examples, without being in any way limited by them.

The three catalysts necessary for the examples were prepared as follows:

A. Preparation of a $ZrO_2$-on-Aerosil-catalyst

68.7 g of $Zr(NO_3)_4 \cdot 5H_2O$ was dissolved in 800 ml of water. The solution was stirred into a suspension of 80 g of Aerosil-380 in 1 litre of water. A solution of 35.9 g of sodium hydroxids in 300 ml of water was then slowly added drop by drop, with vigorous stirring. The resulting white precipitate was filtered off, washed with a few portions of water, dried at 393 K, and calcined in air for 4 h at 723 K. The calcined product was comminuted and sieved to a particle size of 1.20—3.35 mm. The resulting catalyst contained 27.0 wt-% of $ZrO_2$ on Aerosil (and had a bulk density of 440 kg/m).

B. Preparation of a $CeO_2$-on-Aerosil-catalyst

36.5 g of $Ce(NO_3)_3 \cdot 6H_2O$ was dissolved in 200 ml of water. The solution was stirred into a suspension of 56.1 g of Aerosil-380 in 1 litre of water. A solution of 10.1 g of sodium hydroxide in 200 ml of water was then added slowly, with vigorous stirring. The pale violet precipitate was filtered off, washed with water, dried at 383 K, and calcined in air for 4 h at 723 K. The calcined product was comminuted and sieved to a particle size of 1.20—3.35 mm. The resulting catalyst was pale green and contained 17.8 wt-% of $CeO_2$ on Aerosil (and had a bulk density of 320 kg/m³).

C. Preparation of a $ThO_2$-on-α-alumina-catalyst

148 g of α-$Al_2O_3$ granules (2×3 mm) were impregnated with a solution of 110 g of $Th(NO_3)_4 \cdot 4H_2O$ in 50 ml of water. The impregnated granules were dried at 383 K and calcined for 8 h at 723 K. The resulting catalyst contained 26 wt-% of $ThO_2$ on α-alumina and had a bulk density of 2870 kg/m³.

Example I

The tar-like product formed during the decarboxylative oxidation of benzoic acid to phenol was distilled in a distillation column with five plates at a bottom temperature of 533 K, a top temperature of 493 K, a pressure of 33 kPa, and a reflux ratio of 0.5. Benzoic acid was crystallized out of the top stream from the column. The residue was washed with a 20 wt-% solution of sodium hydroxide followed by pure water. A portion of the resulting organic fraction (to be called purified tar) was then vaporized, mixed with nitrogen and steam, and passed at an hourly rate of 19.6 g of purified tar, 68.8 g of nitrogen, and 31.0 g of steam at 723 K and 100 kPa over 50 ml of a $ThO_2$-on-$\alpha$-alumina-catalyst prepared as described above, whereby the purified tar was partly hydrolysed to phenol. The phenol formed was separated from the condensed-out hydrolysis reaction product by distillation. The yield was 13.0 g of phenol per hour.

Example II

A portion of the purified tar, prepared as in Example I, was vaporized, mixed with nitrogen and steam, and passed at an hourly rate of 13.2 g of purified tar, 46.3 g of nitrogen, and 12.0 g of steam at 698 K and 100 kPa over 50 ml of a $ThO_2$-on-$\alpha$-alumina-catalyst prepared as described above, whereby the purified tar was partly hydrolysed to phenol. Phenol was separated from the condensed-out hydrolysis reaction product by distillation. The yield was 9.7 g of phenol per hour.

Example III

A portion of the purified tar, prepared as in Example I, was vaporized, mixed with nitrogen and steam, and passed at an hourly rate of 13.4 g of purified tar, 46.3 g of nitrogen, and 12.0 g of steam at 673 K and 100 kPa over 50 ml of a $ThO_2$-on-$\alpha$-alumina-catalyst prepared as described above, whereby the purified tar was partly hydrolysed to phenol. Phenol was separated from the condensed-out hydrolysis reaction product by distillation. The yield was 9.3 g of phenol per hour.

Example IV

A portion of the purified tar, prepared as in Example I, was vaporized, mixed with nitrogen and steam, and passed at an hourly rate of 20.2 g of purified tar, 68.8 g of nitrogen, and 21.8 g of steam at 723 K and 100 kPa over 50 ml of a $CeO_2$-on-Aerosil-catalyst prepared as described above, whereby the purified tar was partly hydrolysed to phenol. Phenol was separated from the condensed-out hydrolysis reaction product by distillation. The yield was 0.1 g of phenol per hour.

Example V

A portion of the purified tar, prepared as in Example I, was vaporized, mixed with nitrogen and steam, and passed at an hourly rate of 20.8 g of purified tar, 68.8 g of nitrogen, and 21.8 g of steam at 723 K and 100 kPa over 50 ml of a $ZrO_2$-on-Aerosil-catalyst prepared as described above, whereby the purified tar was partly hydrolysed to phenol. Phenol was separated from the condensed-out hydrolysis reaction product by distillation. The yield was 0.3 g of phenol per hour.

## Claims

1. Process for the decarboxylative oxidation of a benzene monocarboxylic acid, preferably benzoic acid, or a salt, ester, or anhydride of such an acid, in the liquid phase at an elevated temperature by means of a molecular oxygen-containing gas, in which process the tar-like product also formed is separated, characterized in that the said tar-like product is subjected to a hydrolysis treatment at a temperature of 500—850 K and a pressure of 50—3000 kPa in the presence of a suitable hydrolysis catalyst consisting at least in part of one or more metals from groups IIIB and IVB of the periodic table of elements, including the rare-earth metals and the actinides, preferably at a load of 0.01—20 kg of the tar-like product per kg of metal in the catalyst per hour.

2. Process in accordance with claim 1, characterized in that the tar-like product is treated with 0.1—10 kg of water and/or steam per kg of the tar-like product.

3. Process in accordance with claim 1 or 2, characterized in that the hydrolysis treatment is carried out at a temperature of 600—800 K.

4. Process in accordance with any of Claims 1—3, characterized in that the hydrolysis treatment is carried out at a pressure of 100—500 kPa.

5. Process in accordance with any of claims 1—4, characterized in that the hydrolysis catalyst consists at least in part of one or more metals chosen from the group consisting of yttrium, cerium, zirconium, hafnium, and/or thorium.

6. Process in accordance with claim 5, characterized in that the catalyst contains thorium, preferably in the form of $ThO_2$.

7. Process in accordance with claim 6, characterized in that the catalyst consists of $ThO_2$ on an $\alpha$-alumina carrier, the quantity of the $ThO_2$ being 20—50 wt-% with respect to the quantity of the $\alpha$-alumina.

8. Process in accordance with any of claims 1—7, characterized in that before the hydrolysis treatment the tar-like product is subjected to a preliminary treatment which at least consists of a distillation step, the said distillation step preferably being accompanied by the introduction of oxygen.

## Patentansprüche

1. Verfahren zur oxydativen Decarboxylierung einer Benzolmonocarbonsäure, vorzugsweise Benzosäure, oder eines Salzes, Esters oder Anhydrids einer solchen Säure, in der flüssigen Phase bei erhöhter Temperatur mittels eines molekularen Sauerstoff enthaltenden Gases, bei welchem Verfahren das gleichfalls gebildete teerartige Produkt abgetrennt wird, dadurch gekennzeichnet, daß das teerartige Produkt einer Hydrolysebehandlung bei einer Temperatur von 500—850 K und einem Druck von 50—3000 kPa in

Gegenwart eines geeigneten Hydrolysekatalysators, bestehend zumindest zum Teil aus einem oder mehreren Metallen aus den Gruppen IIIB und IVB des Periodensystems, einschließlich der Metalle der seltenen Erden und der Actiniden, vorzugsweise bei einer Beladung von 0,01—20 kg des teerartigen Produktes je kg Metall im Katalysator je Stunde unterworfen wird.

2. Verfahren nach Anspruch 1, dadurch gekennzeichnet, daß das teerartige Produkt mit 0,1—10 kg Wasser und/oder Wasserdampf je kg des teerartigen Produktes behandelt wird.

3. Verfahren nach Anspruch 1 oder 2, dadurch gekennzeichnet, daß die Hydrolysebehandlung bei einer Temperatur von 600—800 K durchgeführt wird.

4. Verfahren nach einem der Ansprüche 1—3, dadurch gekennzeichnet, daß die Hydrolysebehandlung bei einem Druck von 100—500 kPa durchgeführt wird.

5. Verfahren nach einem der Ansprüche 1—4, dadurch gekennzeichnet, daß der Hydrolysekatalysator zumindest zum Teil aus einem oder mehreren Metallen, ausgewählt aus der Gruppe Ytrium, Cer, Zirkon, Hafnium und/oder Thorium besteht.

6. Verfahren nach Anspruch 5, dadurch gekennzeichnet, daß der Katalysator Thorium, vorzugsweise in Form von $ThO_2$, enthält.

7. Verfahren nach Anspruch 6, dadurch gekennzeichnet, daß der Katalysator aus $ThO_2$ auf einem α-Aluminiumoxidträger besteht, wobei die Menge des $ThO_2$ 20—50 Gew.-%, bezogen auf die Menge des α-Aluminiumoxids, beträgt.

8. Verfahren nach einem der Ansprüche 1—7, dadurch gekennzeichnet, daß vor der Hydrolysebehandlung das teerartige Produkt einer Vorbehandlung unterzogen wird, welche zumindest aus einer Destillationsstufe, vorzugsweise unter gleichzeitigem Einführen von Sauerstoff, besteht.

## Revendications

1. Procédé d'oxydation décarboxylante d'un acide benzène-monocarboxylique, de préférence l'acide benzoïque ou un sel, ester, ou anhydride d'un tel acide, en phase liquide à une température élevée au moyen d'un gaz contenant de l'oxygène moléculaire, procédé dans lequel on sépare le produit analogue au goudron qui est également formé, caractérisé en ce qu'on soumet ledit produit analogue au goudron à un traitement d'hydrolyse à une température de 500—850 K et une pression de 50—3000 kPa en présence d'un catalyseur d'hydrolyse convenable consistant au moins en partie d'un ou plusieurs métaux des Groupes IIIB et IVB de la Classification Périodique des Eléments, notamment les métaux de terres rares et les actinides, de préférence à une charge de 0,01—20 kg du produit analogue au goudron par kg de métal dans le catalyseur à l'heure.

2. Procédé selon la revendication 1, caractérisé en ce qu'on traite le produit analogue au goudron ave 0,1—10 kg d'eau et/ou de vapeur d'eau par kg du produit analogue au goudron.

3. Procédé selon la revendication 1 ou 2, caractérisé en ce qu'on effectue le traitement d'hydrolyse à une température de 600 à 800 K.

4. Procédé selon l'une quelconque des revendications 1 à 3, caractérisé en ce qu'on effectue le traitement d'hydrolyse sous une pression de 100 à 500 kPa.

5. Procédé selon l'une quelconque des revendications 1 à 4, caractérisé en ce que le catalyseur d'hydrolyse comprend au moins partiellement un ou plusieurs métaux choisis dans le groupe comprenant l'yttrium, le cérium, le zirconium, l'hafnium, et/ou le thorium.

6. Procédé selon la revendication 5, caractérisé en ce que le catalyseur contient du thorium, de préférence sous forme de $ThO_2$.

7. Procédé selon la revendication 6, caractérisé en ce que le catalyseur comprend $ThO_2$ sur un support d'α-alumine, la quantité de $ThO_2$ étant de 20 à 50% pds par rapport à la quantité d'α-alumine.

8. Procédé selon l'une quelconque des revendications 1 à 7, caractérisé en ce qu'avant le traitement d'hydrolyse, on soumet le produit analogue au goudron à un traitement préliminaire qui comprend au moins un stade de distillation, ledit stade de distillation étant accompagné de préférence d'introduction d'oxygène.